# EUROPÄISCHE PATENTSCHRIFT

(11) **EP 0 251 027 B1**
(45) Veröffentlichungstag und Bekanntmachung des Hinweises auf die Patenterteilung: **27.01.1993**
(21) Anmeldenummer: 87108666.6
(22) Anmeldetag: 16.06.1987
(51) Int. Cl.: A61B 5/14

(54) **Vorrichtung zur Bestimmung des Partialdrucks von in einem Fluid gelösten Gasen und Gasgemischen**
Means for measuring the partial pressure of gases and gas mixtures dissolved in a fluid
Dispositif de mesure de la pression partielle de gaz et mélanges de gaz dissous dans un fluide

(30) Priorität: 21.06.1986 DE 3620873
(43) Veröffentlichungstag der Anmeldung: 07.01.1988
(73) Patentinhaber: Rau, Günter, Prof. Dr., D-52066 Aachen (DE); Engelhardt, Harald, Dipl.-Ing., D-52072 Aachen (DE); Reul, Helmut, Prof. Dr., D-52353 Düren (DE)
(72) Erfinder: Rau, Günter, Prof. Dr., D-52066 Aachen (DE); Engelhardt, Harald, Dipl.-Ing., D-52072 Aachen (DE); Reul, Helmut, Prof. Dr., D-52353 Düren (DE)
(74) Vertreter: Münich, Wilhelm, Dr.

(56) Entgegenhaltungen:
- EP-A- 0 000 041
- EP-A- 0 086 338
- GB-A- 2 100 859
- US-A- 4 221 567

## Beschreibung

Die Erfindung bezieht sich auf eine Vorrichtung zur Bestimmung des Partialdrucks von in einem Fluid gelösten Gasen und Gasgemischen, gemäß dem Oberbegriff des Patentanspruchs 1.

Vorrichtungen zur Bestimmung des Partialdrucks von in Fluiden gelösten Gasen und Gasgemischen werden beispielsweise in der Medizin bei der Untersuchung des arteriellen Blutes und der Atemluft verwendet. Weitere Anwendungen derartiger Vorrichtungen liegen beispielsweise im Umweltschutz bei der Stickoxidmessung in Abgasen oder bei der Messung des Sauerstoffgehaltes in stehenden Gewässern. Ein weiteres Beispiel für die Anwendung derartiger Vorrichtungen ist die Messung von Partialdrücken bei Absorptionsvorgängen, beispielsweise bei der Gastrennung und der Gasreinigung.

Abnahme und Analyse arterieller Blutproben stellen den Stand der Technik bei der Messung arterieller O₂- und CO₂-Partialdrücke dar. Die Blutprobenentnahme ist aber immer mit einem Blutverlust für den Patienten verbunden.

Eine Vorrichtung, die mit einem Membranaustauschkatheter arbeitet, ist aus DE-AS 25 34 255 bekannt. Bei dieser Vorrichtung, die insbesondere zur Probenentnahme von im Blut gelösten Gasen bestimmt ist, wird ein Katheter am lebenden Objekt in den zu analysierenden Blutstrom eingeführt. Wenigstens ein Teil des Katheters ist mit einer für die Gase durchlässigen, für das Blut aber im wesentlichen undurchlässigen rohrförmigen Membran versehen, die direkt in Kontakt mit dem Blut steht. Als Trägerfluid wird ein Gas verwendet, das etwa mit Atmosphärendruck in den Katheter eingelassen wird und dabei in Kontakt mit der Membran kommt. Nachdem sich ein Gleichgewicht zwischen dem Trägergas und den im Blut gelösten Gasen eingestellt hat, wird das Gasgemisch aus dem Katheter entfernt und analysiert. Diese bekannte Vorrichtung hat eine Reihe von Nachteilen: Die Verwendung von Gas als Trägerfluid stellt bei Katheterversagen ein gewisses Sicherheitsrisiko dar, weil dann das Trägergas direkt in die Blutbahn gelangen kann. Weiter ist bei der bekannten Vorrichtung lediglich eine diskontinuierliche Messung möglich, da zunächst die Einstellung des Gleichgewichts zwischen dem Trägergas und den im Blut gelösten Gasen abgewartet und erst dann eine Probe aus dem Katheter entnommen wird, die in einer getrennten Einheit analysiert wird. Die Anwendung von gaschromatografischen oder massenspektrometrischen Verfahren zur Analyse des Trägergases bietet sich außerdem mehr im Labor, weniger aber in der klinischen Routine an.

Eine weitere Vorrichtung zur Messung der Blutgaspartialdrücke mit einem Membranaustauschkatheter gemäß dem Oberbegriff des Anspruchs 1 wird in US-A-4 221 567 beschrieben. Bei dieser Vorrichtung wird eine Flüssigkeit durch einen doppellumigen Katheter gepumpt. Das äußere Katheterrohr bildet die gasdurchlässige Membran. Die Partialdrücke in der Trägerflüssigkeit werden bei Verlassen des Katheters gemessen. Die Trägerflüssigkeit wird im Kreislauf umgewälzt, wobei mit einem Kapillarmembranstoffaustauscher definierte Partialdrücke in der Trägerflüssigkeit eingestellt werden, bevor diese erneut in den Katheter eintritt. Eine Kalibrierung dieser Vorrichtung erfolgt dadurch, daß die Umwälzrichtung der Trägerflüssigkeit zyklisch umgekehrt wird, um die Trägerflüssigkeit aus dem Stoffaustauscher unmittelbar in die Meßeinrichtung zurückzuführen.

Der Erfindung liegt die Aufgabe zugrunde, eine Vorrichtung zur Bestimmung des Partialdrucks von in Fluiden gelösten Gasen und Gasgemischen anzugeben, bei der eine kontinuierliche Bestimmung des Partialdrucks ohne Umkehrung der Umwälzrichtung möglich ist.

Diese Aufgabe wird erfindungsgemäß durch die im Patentanspruch 1 angegebenen Merkmale gelöst. Vorteilhafte Weiterbildungen sind in den Unteransprüchen angegeben.

Diese Vorrichtung hat damit den Vorteil, daß sich ohne weiteres eine kontinuierliche Messung der Partialdrücke realisieren läßt, da das Trägerfluid mit den darin gelösten Gasen kontinuierlich durch die Meßeinrichtung strömt.

Die Erfindung geht dabei von dem Grundgedanken aus, daß es nicht erforderlich ist, die Einstellung des Partialdruck-Gleichgewichts zwischen dem Fluid, in dem die Gase bzw. Gasgemische gelöst sind, und dem Trägerfluid abzuwarten. Vielmehr ist es bei einer Vorrichtung, bei der der doppellumig ausgebildete Katheter mit kontinuierlischen Umwalzströmung verwendet wird, ohne weiteres möglich, von dem im Transportfluid gemessenen niedrigen Partialdruck auf den des Fluids zu schließen.

Bei der erfindungsgemäßen Vorrichtung erfolgt nach dem Meßvorgang eine Entreicherung des Trägerfluids von den gelösten Gasen entweder auf einen Partialdruck von nahezu Null oder auf einen bestimmten , vorgebbaren Partialdruck, der beispielsweise gleich dem erwarteten Partialdruck in dem Meßfluid sein kann (Nachführen, Kompensationsmethode).

Deshalb ist es besonders vorteilhaft, daß die erfindungsgemäße Vorrichtung einen Bypass aufweist, durch den entreichertes oder auf einen bestimmten Druck angereichertes Trägerfluid durch die Meßeinrichtung geleitet werden kann (Referenzmessung).

Ein derartiger Bypass kann beispielsweise durch ein Doppel-L-Ventil oder zwei 3/2-Wege-Ventile realisiert werden.

Die erfindungsgemäße Vorrichtung hat zusätzlich zu der Möglichkeit der kontinuierlichen Messung der Partialdrücke ohne Umkehrung der Umwälzrichtung noch eine Reihe von weiteren Vorteilen, die sie insbesondere zur Verwendung in der Blutgas-Analyse prädestinieren:

Insbesondere die Bestimung des arteriellen Sauerstoffpartialdruckes zählt neben der Bestimmung des pH-Wertes und des Basenüberschusses zu den wichtigsten labordiagnostischen Untersuchungen in der klinischen Behandlung respiratorischer und metabolischer Störungen. Der Zusammenhang zwischen dem Sauerstoffpartialdruck und der Sauerstoffkonzentration des Blutes ist durch die Sauerstoffbindungskurve gegeben. Bei bekanntem pH-Wert, Sauerstoff- und Kohlenstoffdioxidpartialdruck lassen sich alle wichtigen Daten des Säure-Basen-Status vollständig ermitteln.

Besondere Ausgestaltungen der erfindungsgemäßen Vorrichtung haben insbesondere beim Einsatz am lebenden Objekt eine Reihe von Vorteilen:

Als Trägerfluid kann eine Flüssigkeit, beispielsweise Wasser, Kochsalzlösung, etc., in der sich das zu bestimmende Gas löst, verwendet werden, so daß sich auch bei Katheter-Versagen kein Sicherheitsrisiko ergibt.

Durch die kontinuierliche Strömung des Trägerfluids durch die Vorrichtung, die insbesondere als geschlossener Kreislauf ausgebildet sein sollte, entfallen sämtliche Sterilisationsprobleme. Das Trägerfluid einschließlich Vorrichtung ist nur einmal vor Einsetzen in die Blutbahn zu sterilisieren. Risiken, die durch das Nachführen neuen Trägerfluids auftreten könnten, entfallen vollständig.

Weiter ist es möglich, das äußere Rohr des Katheters je nach Einsatzfall für alle in dem Fluid gelösten Gase permeabel oder aber selektiv permeabel zu gestalten, um beispielsweise eine "Vorselektion" zu erreichen.

In jedem Fall ist es von Vorteil, wenn das innere Rohr des Katheters gasundurchlässig ist.

Zur Aufrechterhaltung einer Umwälzströmung kann beispielsweise eine Pumpe oder ein Verdichter eingesetzt werden. Natürlich sind auch andere Maßnahmen möglich, mit denen eine kontinuierliche Strömung aufrechterhalten werden kann.

Als Meßeinrichtung können die verschiedensten Einrichtungen zur Bestimmung des Partialdrucks, beispielsweise Massenspektrometer und/oder Gaschromatographen, verwendet werden. Die Vorrichtung ist jedoch besonders für Meßeinrichtungen mit einer nach dem polarographischen Prinzip arbeitenden Elektrode geeignet:

Der Vorteil derartiger polarographischer Elektroden besteht darin, daß sie zuverlässig, einfach zu handhaben und kostengünstig sind und darüberhinaus eine kurze Ansprechzeit haben. Ein weiterer Vorteil ergibt sich daraus, daß bei der Bestimmung des Sauerstoffpartialdruckes der S-förmige Verlauf der Sauerstoffbindungskurve bei arteriellem Blut sehr flach ist. Dies hat zur Folge, daß in diesem Bereich sehr große Änderungen im Sauerstoffpartialdruck nur sehr kleine Änderungen in der Sauerstoffkonzentration des Blutes verursachen. Eine sich anbahnende Mangelversorgung mit Sauerstoff läßt sich also erheblich besser an der Änderung des Partialdruckes feststellen als bei einer direkten Sauerstoff-Konzentrationsbestimmung.

Polarographische Elektroden haben jedoch bei herkömmlichen Vorrichtungen den Nachteil, daß die zu untersuchende Probe gerührt werden muß. Dieses Rühren ist erforderlich, da die Elektrode einen Eigenverbrauch an Sauerstoff hat und sich deshalb in einer unbewegten Probe eine sauerstoffverarmte Zone um die Kathode herum ausbildet. Dieses Phänomen wird allgemein als Stirring-Effekt bezeichnet. Der Stirring-Effekt wird bei der vorliegenden Vorrichtung vermieden, da das Trägerfluid kontinuierlich an der Meßelektrode vorbeiströmt. Eine "Sauerstoffsenke" kann sich also nicht ausbilden.

Die Erfindung wird nachstehend anhand von Ausführungsbeispielen unter Bezugnahme auf die Zeichnungen näher beschrieben. Es zeigen:
- Fig. 1: den Aufbau einer erfindungsgemäßen Vorrichtung zur Bestimmung u.a. des Sauerstoff-Partialdruckes in Blut;
- Fig. 2: das Reaktionsverhalten einer erfindungsgemäßen Vorrichtung auf sprunghafte Partialdruckänderungen im Meßfluid;
- Fig. 3: eine weitere Vorrichtung mit Regeleinrichtung, die nach der Kompensationsmethode arbeitet;
- Fig. 4: eine alternative Ausführung mit zwei gleichzeitig betätigten Umschalteinrichtungen;

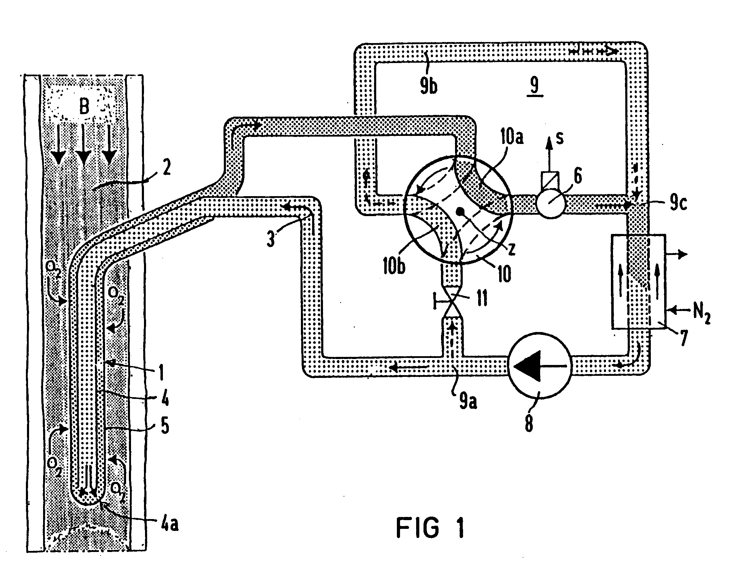

Fig. 1 zeigt ein Ausführungsbeispiel, das insbesondere zur kontinuierlichen intravasalen Partialdruck-Bestimmung von Sauerstoff (pO₂ -Messung) und Kohlenstoffdioxid (pCO₂ -Messung) in einem Fluid wie arteriellem Blut B geeignet ist. In der Vorrichtung ist ein doppellumiger, koaxialer Katheter 1 vorgesehen, der hier in eine Arterie 2 eingesetzt ist und von einem Trägerfluid 3 (Gas oder Flüssigkeit) in einem geschlossenen Kreislauf kontinuierlich durchströmt wird.

Das Trägerfluid 3 ist beispielsweise destilliertes Wasser, das vor dem Eintritt in den Katheter 1 von Sauerstoff O₂ und Kohlenstoffdioxid CO₂ befreit wurde. Es tritt in das innere Rohr 4 des Katheters 1 ein, strömt im inneren Rohr 4 (Lumen) bis zu dessen offenem Ende an der Katheterspitze 4a und fließt im Zwischenraum zwischen dem endseitig geschlossenen äußeren Rohr 5 und dem inneren Rohr 4 zurück. Das aus dem Katheter 1 austretende Trägerfluid 3 fließt über das Wegelement 10a einer als Doppel-L-Schaltventil 10 ausgebildeten ersten und zweiten Umschalteinrichtung zu einer Partialdruck-Meßeinrichtung 6, die neben einer (z.B. polarographischen) Meßeinrichtung für O₂ auch eine Meßeinrichtung für CO₂ enthält. Sie gibt entsprechende Meßsignale s ab. Das Fluid 3 tritt anschließend in eine Stoff-Austauscheinheit 7 ein und wird dort wieder von O₂ und CO₂ befreit. Dazu wird Stickstoff N₂ im Gegenstromprinzip durch die Einheit 7 geleitet; man könnte auch Luft verwenden. Das Trägerfluid 3 wird anschließend von einem Verdichter oder einer Pumpe 8 in das innere Rohr 4 des Katheters 1 zurückgepumpt.

Die Sauerstoff-Austauscheinheit 7 kann auch so ausgebildet sein, daß in ihr - anstelle einer vollständigen Befreiung von O₂ und CO₂ - eine Anreicherung oder eine Abreicherung auf einen jeweils vorgegebenen Partialdruck vorgenommen wird. Dies ist insbesondere für die später beschriebene "Kompensationsmethode" von Bedeutung.

Weiter weist die Vorrichtung einen allgemein mit 9 bezeichneten Bypass auf. Dieser Bypass 9 führt von einem ersten T-Stück 9a am Ausgang der Pumpe 8 über ein Feindosierventil 11, ein Wegeelement 10b des Doppel-L-Schaltventils 10 und ein Leitungsstück 9b zu einem zweiten T-Stück 9c am Eingang der Stoff-Austauscheinheit 7. Bei der gezeigten (durchgezogen gezeichneten) Schaltdarstellung I des Doppel-L-Schaltventils 10 ergibt sich also ein Bypass für das in der Stoff-Austauscheinheit 7 behandelte Trägerfluid 3 über das Feindosierventil 11. Dieser Fluidfluß ist durch vier gestrichelte Pfeile markiert. Das von der Pumpe 8 geförderte Trägerfluid 3 wird also aufgeteilt: Ein Teil fließt über den Katheter 1 und ein anderer Teil über den Bypass 9. Das Feindosierventil 11 wird einmal von Hand eingestellt und bleibt dann in dieser (geöffneten) Stellung stehen. Auf diese Weise wird die Strömungsgeschwindigkeit des Trägerfluids 3 im Katheter 1 eingestellt.

Im folgenden sollen der Aufbau und die Funktion der vorstehend vorgestellten Elemente des in Fig. 1 dargestellten Ausführungsbeispiels erläutert werden:

Der doppellumige Katheter 1 hat bei der intravasalen Partialdruckmessung in arteriellem Blut B beispielsweise einen Außendurchmesser vom 1,9 mm. Das innere Rohr 4 wird von einem Polytetrafluoräthylen-Schlauch gebildet, der für die zu messenden Blutgase, d.h. für Sauerstoff O₂ und Kohlenstoffdioxid CO₂, undurchlässig ist. Das äußere Rohr 5 besteht bevorzugt in seiner gesamten, in die Arterie 2 eingeführten Länge aus einem Werkstoff, der eine gute Durchlässigkeit für die Blutgase, nicht aber für das Blut B aufweist, z.B. Siliziumkautschuk oder Polypropylen. Es ergibt sich somit eine relativ große Austauschfläche für diese Gase. Beim Einführen des Katheters 1 in das sauerstoffreiche arterielle Blut B können damit Sauerstoff O₂ und Kohlendioxid CO₂ aus dem Blut B durch den äußeren Schlauch 5 in das den Katheter 1 durchströmende Trägerfluid 3 eindiffundieren, das damit angereichert wird.

Die Meßeinrichtung 6 ist in an sich bekannter Weise aufgebaut. Sie kann einen in die Wand eines Rohrstücks eingebauten O₂- und CO₂-Sensor aufweisen. Für die Sauerstoffmessung kann sie als polarographische Meßeinrichtung aufgeführt werden. Mit ihr wird die Anreicherung des Trägerfluids 3 bestimmt. Aus dem Maß der Anreicherung wird auf den Sauerstoff- und Kohlendioxidpartialdruck in dem den Katheter 1 umgebenden Blut B geschlossen. Die Vorrichtung unterscheidet sich dabei von bekannten Vorrichtungen darin, daß sich nicht ein Gleichgewicht, sondern lediglich stationäre Verhältnisse einstellen müssen, um so nach kurzer Wartezeit eine Information über den Gas-Partialdruck des Blutes B zu erhalten. Unter dem Begriff "Gleichgewicht" wird dabei die Gleichheit des Partialdrucks von Trägerfluid 3 und zu messendem Fluid Blut B verstanden; und als "stationäres Verhältnis" wird eine zeitliche Konstanz des Partialdrucks des Trägerfluids 3 angesehen.

Die Stoff-Austauscheinheit 7 ist bei dem in Fig. 1 gezeigten Ausführungsbeispiel ein Kapillarrohr-Stoffaustauscher, durch den die angereicherte Trägerflüssigkeit 3 gepumpt wird. Er wird im Gegenstromprinzip von Stickstoff (N₂) durchströmt, vgl. Pfeile in der Einheit 7. Die Trägerflüssigekeit 3 gibt in der Austauscheinheit 7 den in ihr gelösten Sauerstoff und das gelöste Kohlenstoffdioxid wieder ab und nimmt statt dessen Stickstoff N₂ auf.

Das Schaltventil 10 kann von der gezeigten Meßstellung (Schaltstelung I) um 90° um den Punkt Z gedreht werden und damit in die gestrichelt gezeichnete Bypass-Stellung (Schaltstellung II) umgeschaltet werden. Der Bypass 9 ermöglicht es nun, vor dem Katheter 1 über das T-Stück 9a einen Flüssigkeitsteilstrom abzuzweigen und diesen über das Dosierventil 11 und das in Schaltstellung II befindliche Doppel-L-Schaltventil 10 direkt in die Meßeinrichtung 6 zu leiten. Der Teilstrom strömt über die Elemente 8, 9a, 11, 10b, 6, 9c und 7. Hierdurch ergibt sich die Möglichkeit, die Partialdrücke der Trägerflüssigkeit 3 vor und nach der An- bzw. Entreicherung zu überprüfen. Damit ist es möglich, eine Referenzmessung durchzuführen, d.h. zu überprüfen, ob z.B. die Entreicherung der Trägerflüssigkeit 3 von Sauerstoff und Kohlenstoffdioxid in der Stoff-Austauschereinheit 7 hinreichend vollzogen worden ist. Zur Durchführung der Referenzmessung wird der Volumenstrom durch den Bypass 9 mittels des Feindosierventils 11 (z.B. gleich dem durch den Katheter 1) eingestellt.

Die Meßeinrichtung 6 weist bei dem beschriebenen Ausführungsbeispiel bei der Sauerstoff-Messung bevorzugt eine polarographische Elektrode auf. Für die CO₂-Messung kann bevorzugt eine Glaselektrode verwendet werden. Beide Elektroden können parallel oder in Serie durchströmt werden. Bei Bedarf können weitere Elektroden, z.B. für Elektrolyte wie Kalium-, Natrium-oder Calcium-Verbindungen, vorgesehen sein. Der bei der Messung in der polarographischen Elektrode reduzierte Strom ist bekanntlich proportional zum Partialdruck des Sauerstoffs in der Trägerflüssigkeit 3. Läßt man beispielsweise Luft anstelle von Stickstoff in der gestrichelten Bypass-Stellung II des Schaltventils 10 durch die Austauschereinheit 7 strömen, so kann man die Elektrodenmeßanordnung kalibrieren. Durch Festlegen des Nullpunktes und des durch Luftäquilibrierung eingestellten Meßpunktes kann man die Empfindlichkeit der Elektroden, z.B. auch das lineare Verhalten des Meßsignals s bezügl. der Meßgröße, einfach überprüfen.

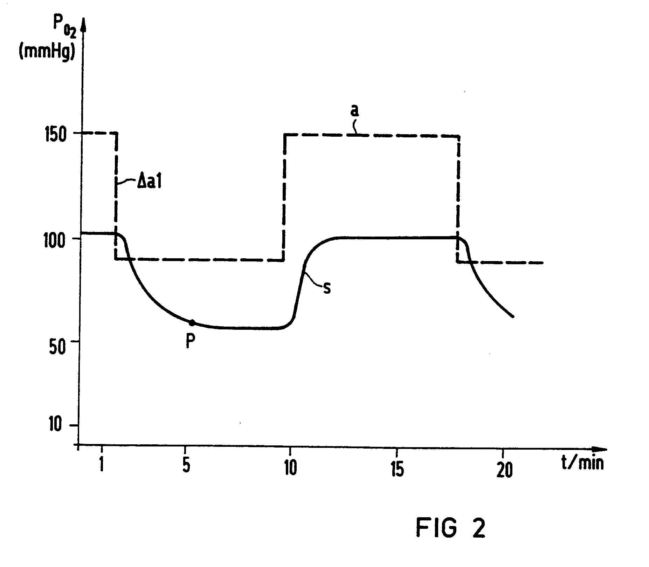

Fig. 2 zeigt das Reaktionsverhalten der in Fig. 1 dargestellten Vorrichtung auf Änderungen des Sauerstoffpartialdrucks in einem Meßfluid B. Das Elektrodenausgangssignal s ist mit einem x/t-Schreiber als Funktion der Zeit t (in Minuten) aufgezeichnet. Das Elektrodenausgangssignal s ist dabei direkt in Druckeinheiten, und zwar in mm Hg, kalibriert. Das Signal s entspricht damit dem Sauerstoff-Partialdruck pO₂.

In Fig. 2 sind zusätzlich sprungförmig aufgeprägte Partialdruckänderungen in einer gestrichelten Kurve a eingezeichnet. Ein Vergleich zeigt: Eine sprungförmig hervorgerufene Änderung des Partialdrucks, z.B. eine Änderung Δ al in dem den Katheter 1 umgebenden Medium B von der Größe der Sauerstoffpartialdruckdifferenz zwischen arteriellem und venösem Blut, wird nach etwa 15 sec an der Elektrode (Meßeinrichtung 6) registriert. Der Endwert nach Erreichen stationärer Verhältnisse wird nach etwa 3 Minuten in der Trägerflüssigkeit 3 erreicht; dies ist durch einen Punkt P gekennzeichnet. Etwa ab dem Punkt P liegen stationäre Verhältnisse vor. Damit ist der Partialdruck in dem den Katheter 1 umströmenden Fluid 3 quantitativ als Meßwert bestimmt.

Ein deutlicher Partialdruckabfall beispielsweise von Sauerstoff O₂ kann damit schon nach etwa 15 sec an der Partialdruckänderungsgeschwindigkeit abgelesen werden. Hieraus kann ein Alarm, z.B. für die Patientenüberwachung, oder ein Signal für andere Zwecke gebildet werden. Folglich bietet sich beispielsweise für einen Anästhesisten schon zu diesem Zeitpunkt die Möglichkeit einer Gegenmaßnahme. Andererseits läßt sich anhand der ermittelten Partialdruckänderungsgeschwindigkeit auch ein Trend des Blutgas-Partialdrucks, z.B. eine sich anbahnende Mangelversorgung des Patienten, leicht ablesen.

Vorstehend ist die Erfindung anhand eines Ausführungsbeispiels anhand der Figuren 1 und 2 beschrieben worden. Innerhalb des allgemeinen Erfindungsgedankens, nämlich einen doppellumig durchströmten Katheter 1 zu verwenden und den Partialdruck der in dem Trägerfluid 3 gelösten Gase zu bestimmen, ohne die Einstellung eines Gleichgewichts abzuwarten, sind selbstverständlich verschiedene Modifikationen möglich: Anstelle von destilliertem Wasser als Trägerfluid 3 können selbstverständlich auch andere Flüssigkeiten oder Gase verwendet werden. Erforderlich ist lediglich, daß sich die Gase, deren Partialdruck bestimmt werden soll, in dem Trägerfluid 3 lösen.

Die Verwendung eines Gases als Trägerfluid 3 hat den Vorteil, daß sich ein noch schnelleres Reaktionsverhalten der Vorrichtung ergibt als bei einer Flüssigkeit, da die Diffusion in Gasen erheblich schneller abläuft als in Flüssigkeiten.

Wenn als Trägerfluid ein Gas verwendet wird, kann die beschriebene Vorrichtung beispielsweise als hochempfindliches, schnell reagierendes System zur Stickoxidmessung in Abgasen eingesetzt werden.

In der Medizintechnik und insbesondere bei Messungen in Adern ist es jedoch aus Sicherheitsgründen (Undichtigkeit des Katheters 1, z.B. durch Beschädigung) sowie aus Gründen der Miniaturisierung (Verwendung einer (Flüssigkeits-)Pumpe anstelle eines (Gas-)Verdichters) von Vorteil, als Trägerfluid eine Flüssigkeit einzusetzen. Weiter ist es in der Medizintechnik von Vorteil, daß die Vorrichtung einen geschlossenen Kreislauf aufweist, da sich dann Sterilisierungsprobleme etc. nicht stellen. In anderen Einsatzbereichen, beispielsweise beim Umweltschutz, kann es von Vorteil sein, zusätzlich eine ferngesteuerte Spüleinrichtung vorzusehen, damit Kontaminationen beseitigt werden können. Das Meßsystem kann damit wartungsfrei beispielsweise an schwer zugänglichen Meßstellen eingesetzt werden.

Die Verwendung einer nachgeschalteten Auswerteeinheit, die beispielsweise Trends des Partialdrucks erkennen kann, ermöglicht die Integration der Vorrichtung beispielsweise in ein automatisiertes Regelsystem in der Dialyse, bei der künstlichen Beatmung und der extracorporalen CO₂-Entsorgung.

Weiter ist es möglich, die Strömungsrichtung durch den Katheter 1 umzukehren. Weiterhin kann das äußere Rohr 5 nicht für alle Gase, sondern selektiv für bestimmte Gase permeabel sein. Dies gilt beides für die gesamte Länge des in das Fluid untergebrachten Rohres 5. Das obere Ende kann allerdings aus einem für Gase undurchdringlichen Werkstoff ausgebildet sein. Auch das innere Rohr 4 kann unter bestimmten Umständen selektiv, es kann aber auch integral permeabel ausgeführt sein.

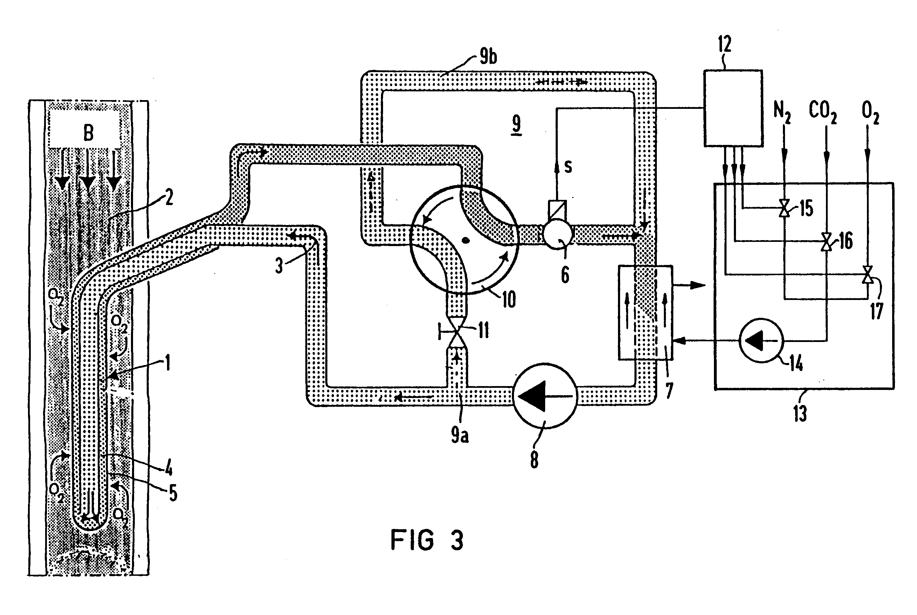

In Figur 3 ist ein Ausführungsbeispiel gezeigt, das nach einer "Kompensationsmeßmethode" arbeitet. Alternativ zu Stickstoff N₂ kann nämlich in der "Kompensationsmeßmethode" die Stoff-Austauscheinheit 7 von einem Gasgemisch aus insbesondere O₂, CO₂ und N₂ durchströmt werden, das (in etwa) den gleichen Sauerstoff- und Kohlendioxidpartialdruck hat wie das zu messende Fluid (Blut B).

Der Meßaufbau zur "Kompensationsmeßmethode" nach Fig. 3 enthält einige Bauelemente zusätzlich zu Fig. 1. Bei der "Kompensationsmeßmethode" ist eine Regelung erforderlich, da ein gemessener Wert (Istwert, bestimmt durch das Gasgemisch) mit einem angestrebten Wert (Sollwert, im Fluid gemessen) verglichen wird, und da der Istwert dem Sollwert ständig angenähert oder nachgeführt wird.

Die Kompensation bei der Partialdruckmessung besteht darin, daß der Sauerstoff- und Kohlendioxidpartialdruck des Trägerfluids 3 dem des zu messenden Fluids Blut B angeglichen wird.

Der O₂-, CO₂-Partialdruck des Blutes B ist also der Sollwert. Er wird in Meßstellung des Doppel-L-Schaltventils 10, also in der gezeigten Schaltstellung I, bestimmt und gespeichert. Der Istwert ist der O₂-, CO₂-Partialdruck des Trägerfluids 3. Er wird danach in Bypassstellung des Doppel-L-Schaltventils 10, also in der um 90° gedrehten Schaltstellung II, bestimmt und gespeichert. Für beide Messungen wird also dieselbe Meßeinrichtung 6 herangezogen. Der Vergleich zwischen Ist- und Sollwert erfolgt in einem Regler 12.

Da in der Meßeinrichtung nur noch Abweichungen zwischen Soll-und Istwert gemessen werden müssen und sich die Abweichungen in einem wesentlich kleineren Rahmen als die Absolutwerte bewegen, ist mit der "Kompensationsmeßmethode" eine größere Meßgenauigkeit zu erzielen.

Zur Realisierung der "Kompensationsmeßmethode" wird eine Gasmischpumpe 13 verwendet, die ausgangsseitig die Stoffaustauschereinheit 7 mit der jeweils neu einzustellenden Gaszusammensetzung versorgt ("identische Beladung"). Die Gasmischpumpe 13 hat Anschlüsse an eine Stickstoff- (N₂-), Sauerstoff- (O₂-) und eine Kohlendioxid- (CO₂-) Versorgung. Sie enthält das eigentliche Pumpaggregat 14 und Stellglieder 15, 16, 17 in Form von Ventilen. Diese Stellglieder 15, 16, 17 werden von dem Regler 12, der wiederum von den Signalen s (Istwert, Sollwert) der Meßeinrichtung 6 beaufschlagt ist, mit Stellsignalen belegt. Wie bereits dargelegt: in der Meßeinrichtung 6 werden Sollwert und Istwert über die Meßelektroden für O₂ und CO₂ bestimmt und über den Regler 18 für die Steuerung der Gasmischpumpe 13 verwendet.

Wird z.B. von der Sauerstoffelektrode ein steigender Sauerstoffpartialdruck im Blut B registriert, so wird im Regler 12 dieser Wert mit dem Istwert im Trägerfluid 3 verglichen und bei einer Abweichung ein Signal an die Gasmischpumpe 13 weitergegeben. Ist beispielsweise der Istwert im Trägerfluid 3 zu niedrig, so wird über die Stellglieder 17 und 15 z.B. die Sauerstoffzufuhr erhöht und die Stickstoffzufuhr gedrosselt. Dies geschieht innerhalb der Gasmischpumpe 13 z.B. über Proportional- und Servoventile. Gasmischpumpen für diese Zwecke sind auf dem Markt erhältlich.

Diese Variante nach der "Kompensationsmeßmethode" wird auch als geschlossenes Kreislaufsystem und damit kontinuierlich betrieben.

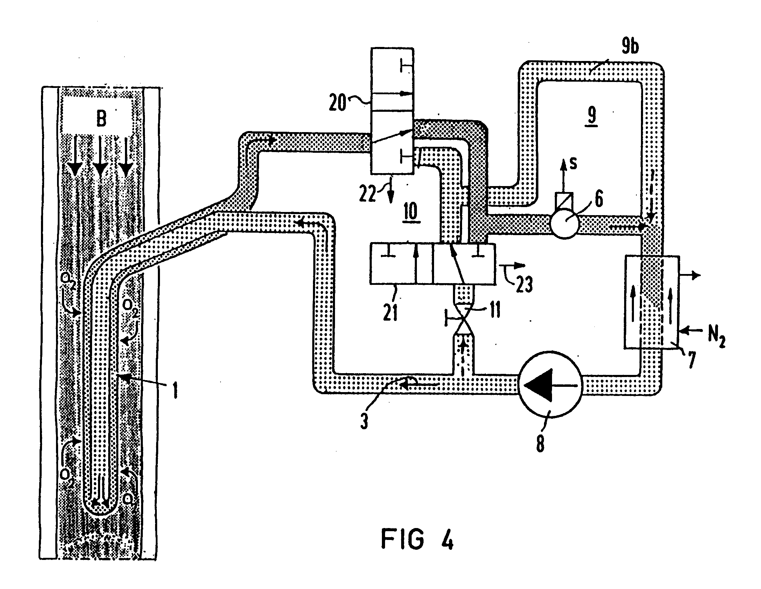

Die Realisierung des Bypasses 9 kann auch anders als bisher beschrieben erfolgen: Nach Fig. 4 kann beispielsweise das in Pfeilrichtung umschaltbare Doppel-L-Schaltventil 10 durch zwei über Kreuz geschaltete 3/2-Wege-Ventile 20, 21 ersetzt werden. Solche 3/2-Wege-Ventile 20, 21 sind an sich bekannt und auf dem Markt erhältlich (z.B. von der Fa. WABCO Westinghouse Steuerungstechnik GmbH & Co., D-3000 Hannover). Aus der gezeigten Schaltstellung I läßt sich die Vorrichtung durch gleichzeitiges Schalten in Richtung der Pfeile 22, 23 in die Schaltstellung II überführen. Dies kann anhand der in den Ventilen 20, 21 angegebenen Symbole leicht verfolgt werden. Die beiden Ventile 20, 21 repräsentieren eine erste bzw. eine zweite Umschalteinrichtung.

Wie früher ausgeführt, kann die Durchflußmeßeinrichtung 6 durch ein Rohrstück gebildet werden, in dessen Wand handelsübliche Sensoren für O₂ und CO₂ eingelassen sind. Ein solches Rohrstück kann für jeden Sensor also T-förmig ausgebildet sein.

## Patentansprüche

1. Vorrichtung zur Bestimmung des Partialdrucks eines in einem Fluid (B) gelösten Gases, bei der in das Fluid (B) ein Katheter (1) einsetzbar ist, der ein Trägerfluid (3) enthält und dessen Katheterwand für das gelöste Gas permeabel ist, mit einer Meßeinrichtung (6) zur Bestimmung des Partialdrucks des Gases im Trägerfluid (3), wobei der Katheter ein doppellumiger koaxialer Katheter (1) ist, dessen äußeres Rohr (5) für das Gas permeabel ist, und eine Einrichtung (8) vorgesehen ist, die eine Strömung des Trägerfluids (3) durch das innere Rohr (4), durch den Zwischenraum zwischen dem äußeren und dem inneren Rohr (4, 5), durch die Meßeinrichtung (6) für den Partialdruck und durch eine Stoff-Austauscheinheit (7), in dem das angereicherte Trägerfluid (3) auf einen bestimmten Partialdruck des Gases gebracht wird, oder umgekehrt, aufrechterhält, **dadurch gekennzeichnet,** daß ein Bypass (9) vorgesehen ist, durch den ein Teilstrom des die Stoff-Austauscheinheit verlassenden Trägerfluids (3) strömt und in die Meßeinrichtung (6) geleitet wird., So daß eine Referenzmessung bei kontinuierlischen Strömung des Trägerfluids in einer einzigen Richtung erfolgen kann.

2. Vorrichtung nach Anspruch 1, **dadurch gekennzeichnet,** daß das äußere Rohr (5) des Katheters (1) für mehrere oder alle in dem Fluid (B) gelösten Gase permeabel ist.

3. Vorrichtung nach Anspruch 1, **dadurch gekennzeichnet**, daß das äußere Rohr (5) des Katheters (1) selektiv permeabel ist.

4. Vorrichtung nach einem der Ansprüche 1 bis 3, **dadurch gekennzeichnet,** daß das innere Rohr (4) von einem Polytetrafluoräthylen. Schlauch gebildet ist.

5. Vorrichtung nach einem der Ansprüche 1 bis 4, **dadurch gekennzeichnet,** daß das innere Rohr (4) für das gelöste Gas undurchlässig ist.

6. Vorrichtung nach einem der Ansprüche 1 bis 5, **dadurch gekennzeichnet,** daß die Einrichtung (8) dazu vorgesehen ist, einen geschlossenen Kreislauf des Trägerfluids (3) aufrechtzuerhalten.

7. Vorrichtung nach einem der Ansprüche 1 bis 6, **dadurch gekennzeichnet,** daß das Trägerfluid (3) eine Flüssigkeit ist.

8. Vorrichtung nach einem der Ansprüche 1 bis 7, **dadurch gekennzeichnet,** daß als Einrichtung zur Aufrechterhaltung der Strömung des Trägerfluids (3) eine Pumpe (8) oder ein Verdichter vorgesehen ist, die/der -in Strömungsrichtung gesehen - vorzugsweise hinter der Stoff-Austauscheinheit (7) angeordnet ist.

9. Vorrichtung nach einem der Ansprüche 1 bis 8, **dadurch gekennzeichnet,** daß die Meßeinrichtung (6) für den Partialdruck des gelösten Gases eine polarographische Elektrode und/oder eine Glaselektrode aufweist.

10. Vorrichtung nach Anspruch 7, dadurch gekennzeichnet, daß die Flüssigkeit eine Kochsalzlösung ist

11. Vorrichtung nach einem der Ansprüche 1 bis 10, **dadurch gekennzeichnet,** daß der Bypass (9) durch zwei T-Stücke (9a, 9c) und ein Dosierventil (11) gebildet ist.

12. Vorrichtung nach Anspruch 11, **dadurch gekennzeichnet,** daß eine erste Umschalteinrichtung (10) vorgesehen ist, mit deren Hilfe das aus dem Katheter (1) strömende Trägermedium (3) wahlweise der Meßeinrichtung (6) oder der Stoff-Austauscheinheit (7) zuleitbar ist.

13. Vorrichtung nach einem der Ansprüche 11 bis 12, **dadurch gekennzeichnet,** daß eine zweite Umschalteinrichtung (10) vorgesehen ist, mit deren Hilfe das aus der Stoff-Austauscheinheit (7) strömende Trägermedium (3) wahlweise direkt der Stoff-Austauscheinheit (7) oder der Meßeinrichtung (6) zuleitbar ist.

14. Vorrichtung nach Anspruch 12 und 13, **dadurch gekennzeichnet,** daß sich in einer ersten Schaltstellung (I) der Umschalteinrichtungen (10) ein Strömungsweg von der Strömungsaufrechterhaltungs-Einrichtung (8) über ein Dosierventil (11) und die Stoff-Austauscheinheit (7) zurück zu dieser Einrichtung (8) und in einer zweiten Schaltstellung (11) der Umschalteinrichtungen (10) ein Strömungsweg von der Strömungsaufrechterhaltungs-Einrichtung (8) über das Dosierventil (11), die Meßeinrichtung (6) und die Stoff-Austauscheinheit (7) zurück zu dieser Einrichtung (8) ergibt.

15. Vorrichtung nach Anspruch 14, **dadurch gekennzeichnet,** daß ein Doppel-L-Ventil (10) oder zwei über Kreuz geschaltete 3/2-Wege-Ventile (20, 21) die Umschalteinrichtungen (10) bilden.

16. Vorrichtung nach einem der Ansprüche 1 bis 15, **dadurch gekennzeichnet,** daß an die Stoff-Austauscheinheit (7) eine Gasmischpumpe (13) angeschlossen ist, die von verschiedenen Gasen (N₂, CO₂, O₂) gespeist und von Stellsignalen aus einem Regler (12) beaufschlagt ist.

17. Vorrichtung nach den Ansprüchen 11, 12 und 16, **dadurch gekennzeichnet,** daß die Meßeinrichtung (6) in einer ersten Schaltstellung (I) zur Bestimmung des Sollwerts und in einer zweiten Schaltstellung (II) zur Bestimmung des Istwerts für den Regler (12) vorgesehen ist.

18. Vorrichtung nach einem der Ansprüche 1 bis 17, **dadurch gekennzeichnet,** daß die Stoff-Austauscheinheit (7) eine Entreicherungseinrichtung ist.

19. Vorrichtung nach einem der Ansprüche 1 bis 18, **dadurch gekennzeichnet,** daß es zur kontinuierlichen intravasalen Partialdruck-Bestimmung von Sauerstoff und Kohlenstoffdioxid in arteriellem Blut (B) vorgesehen ist.

## Claims

1. Device for determining the partial pressure of a gaz dissolved in a fluid (B), wherein a catheter (1) is adapted to be inserted into the fluid (B), which contains a carrier fluid (3) and whose catheter wall is permeable to the dissolved gas, comprising a measuring means (6) for determining the partiel pressure of the gas in said carrier fluid (3), with said catheter being a coaxial catheter (1) having two lumina, whose outer tube (5) is permeable to the gas, and means (8) being provided for maintaining a flow of said carrier fluid (3) through the inner tube (4), through the space between said outer and inner tubes (4, 5), through said measuring means (6) for determining the partial pressure, and through a substance exchanger (7) where the enriched carrier fluid (3) is carried to a defined partial pressure of the gaz or vice versa,
**characterized in** that a by-pass (9) is provided through which flows a partial flow of the carrier fluid (3) leaving said substance exchanger, which flow is introduced into said measuring means (6).

2. Device according to Claim 1,
**characterized in** that said outer tube (5) of said catheter (1) is permeable to several or all of the gases dissolved in the fluid (B).

3. Device according to Claim 1,
**characterized in** that aid outer tube (5) of said catheter (1) is selectively permeable .

4. Device according to any of Claims 1 to 3,
**characterized in** that said inner tube (4) is formed by a flexible tubing of tetrafluorethylene

5. Device according to any of Claims 1 to 4,
**characterized in** that said inner tube (4) is impermeable to the dissolved gas.

6. Device according to any of Claims 1 to 5,
**characterized in** that said means (8) are provided to maintain a circulation of said carrier fluid (3) in a closed loop.

7. Device according to any of Claims 1 to 6,
**characterized in** that said carrier fluid (3) is a liquid.

8. Device according to any of Claims 1 to 7,
**characterized in** that a pump (8) or a compressor is provided as means for maintaining the flow of said carrier fluid (3), which - seen in the flow direction - is preferably disposed behind said substance exchanger (7).

9. Device according to any of Claims 1 to 6,
**characterized in** that said measuring means (6) for determining the partial pressure of the dissolved gas comprises a polarographic electrode and/or a glass electrode.

10. Device according to Claim 7,
**characterized in** that aid liquid is a saline of common salt.

11. Device according to any of Claims 1 to 10,
**characterized in** that said by-pass (9) is formed by two T-elements (9a, 9c) and one dosing valve (11).

12. Device according to Claim 11,
**characterized in** that a first change-over means (10) is provided which may be used to feed the carrier medium (3) leaving said catheter (1) optionally to said measuring means (6) or said substance exchanger (7).

13. Device according to any of Claims 11 to 12,
**characterized in** that a second changeover means (10) is provided which may be used to feed the carrier medium (3) leaving said substance exchanger (7) optionally directly to said substance exchanger (7) or said measuring means (6).

14. Device according Claims 12 and 13,
**characterized in** that with a first control position (I) of said change-over means (10) is established a flow path from said flow-maintaining means (8) through a dosing valve (11) and said substance exchanger (7) back to these means (8) while with a second control position (II) of said change-over means (10) is established a flow path from said flow-maintaining means (8) through said dosing valve (11), said measuring means (6) and said substance-exchanger (7) back to these means (8).

15. Device according to Claim 14,
**characterized in** that a double-L valve (10) or two 2/3-way valves (20, 21) disposed at right angles constitute said change-over means (10).

16. Device according to any of Claims 1 to 15,
**characterized in** that a gas mixer pump (13) is connected to said substance exchanger (7), which is supplied with various gases (N₂, CO₂, O₂) and controlled by adjusting signals from a regulator (12).

17. Device according to Claims 11, 12 and 16,
**characterized in** that said measuring means (6) is provided to determine the set value in a first control position (I) and for determining the actual value for said regulator (12) in a second control position.

18. Device according to any of Claims 1 to 17,
**characterized in** that aid substance exchanger (7) is a depleting means.

19. Device according to any of Claims 1 to 18,
**characterized in** that it is intended for continuous intravasal partial pressure determination of oxygen and carbon dioxide in artial blood (B).

## Revendications

1. Dispositif pour déterminer la pression partielle d'un gaz dissou dans un fluide (B), dans lequel on peut introduire dans le fluide (B) un cathéter (1) qui contient un fluide porteur (3) et dont la paroi de cathéter est perméable pour le gaz dissou, comprenant des moyens de mesure (6) pour déterminer la pression partielle du gaz dans ledit fluide porteur (3), ledit cathéter étant un cathéter coaxial à deux volumes (1) dont le tube extérieur (5) est perméable au gaz, et des moyens (8) étant prévus pour maintenir un courant dudit fluide porteur (3) par le tube intérieur (4), par l'espace entre le tube extérieur et le tube intérieur (4, 5), par les moyens de mesure (6) de la pression partielle et par un échangeur de matières (7) où le fluide porteur (3) enrichi est porté à une pression partielle définie du gaz, ou vice versa,
**caractérisé** en ce qu'une dérivation (9) est prévue par laquelle s'écoule un courant partiel du fluide porteur (3) venant de l'échangeur de matières (7) et est introduit dans les moyens de mesure (6), de façon qu'un mesurage de référence soit réalisable à écoulement du fluide porteur en continu suivant un seul sens.

2. Dispositif selon la revendication 1,
**caractérisé** en ce que ledit tube extérieur (5) du cathéter (1) est perméable pour plusieurs ou tous les gaz dissous dans ledit fluide (B).

3. Dispositif selon la revendication 1,
**caractérisé** en ce que ledit tube extérieur (5) du cathéter (1) est d'une perméabilité sélective.

4. Dispositif selon une quelconque des revendications 1 à 3,
**caractérisé** en ce que ledit tube intérieur (4) est formé par un tuyau en polytétrafluoréthylène.

5. Dispositif selon une quelconque des revendications 1 à 4,
**caractérisé** en ce que ledit tube intérieur (4) est imperméable audit gaz dissou.

6. Dispositif selon une quelconque des revendications 1 à 5,
**caractérisé** en ce que lesdits moyens (8) sont prévus pour maintenir une circulation du fluide porteur (3) en circuit fermé.

7. Dispositif selon une quelconque des revendications 1 à 6,
**caractérisé** en ce que ledit fluide porteur (3) est un liquide.

8. Dispositif selon une quelconque des revendications 1 à 7,
**caractérisé** en ce que'une pompe (8) oiu un compresseur est prévu en tant que moyens pour maintenir l'écoulement du fluide porteur (3), laquelle pompe ou lequel compresseur est disposé de préférence à la suite dudit échangeur de matières (7), vu en sens d'écoulement.

9. Dispositif selon une quelconque des revendications 1 à 8,
**caractérisé** en ce que lesdits moyens de mesure (6) de la pression partielle du gaz dissou comprend une électrode polarographique et/ou une électrode en verre.

10. Dispositif selon la revendication 7,
**caractérisé** en ce que ledit fluide est une solution de sel de cuisine.

11. Dispositif selon une quelconque des revendications 1 à 10,
**caractérisé** en ce que ladite dérivation (9) est formée par deux pièces en T (9a, 9c) et une vanne de dosage (11).

12. Dispositif selon la revendication 11,
**caractérisé** en ce que des premiers moyens de changement (10) sont prévus au moyen desquels ledit milieu porteur (3) sortant du cathéter (1) se peut alimenter, à volonté, auxdits moyens de mesure (6) ou l'échangeur des matières (7).

13. Dispositif selon une quelconque des revendications 11 à 12,
**caractérisé** en ce que des deuxièmes moyens de changement (10) sont prévus au moyen desquels ledit milieu porteur (3) sortent de l'échangeur de matières (7) se peut alimenter, à volonté, directement à l'échangeur de matières (7) ou lesdits moyens de mesure (6).

14. Dispositif selon les revendications 12 et 13,
**caractérisé** en ce qu'à une première position de commande (I) des moyens de changement (10) il en résulte une voie d'écoulement desdits moyens (8) pour maintenir le courant par une vanne de dosage (11) et ledit échangeur des matières (7) et retour auxdits moyens (8), et à une deuxième position de commande (II) desdits moyens de changement (10) il en résulte une vole d'écoulement desdits moyens (8) pour maintenir le courant par une vanne de dosage (11), lesdits moyens de mesure (6) et ledit échangeur des matières (7) et retour auxdits moyens (8).

15. Dispositif selon la revendication 14,
**caractérisé** en ce qu'une vanne en L double (10) ou deux vannes à 3/2 voles (20, 21) disposées à se croiser constituent lesdits moyens de changement (10).

16. Dispositif selon une quelconque des revendications 1 à 15,
**caractérisé** en ce qu'une pompe mélangeuse de gaz (13) est raccordée audit échangeur de matières (7), qui est alimentée en gaz différents (N₂, CO₂, O₂) et commandée par des signaux de réglage qui proviennent d'un régulateur (12).

17. Dispositif selon les revendications 11, 12 et 16,
**caractérisé** en ce que lesdits moyens de mesure (6) sont prévus pour déterminer la valeur théorique à une première position de commande (I) et à une deuxième position de commande (II) pour déterminer la valeur réelle pour ledit régulateur (12)

18. Dispositif selon une quelconque des revendications 1 à 17,
**caractérisé** en ce que ledit échangeur de matières (7) est un dispositif d'appauvrissement.

19. Dispositif selon une quelconque des revendications 1 à 18,
**caractérisé** en ce qu'il est destiné à la détermination de la pression partielle intravasale d'oxygène et dioxyde de carbon en continu dans un courant de sang artériel.
